# EUROPEAN PATENT APPLICATION

(11) **EP 2 119 695 A1**
(43) Date of publication of application: **18.11.2009**
(21) Application number: 08009046.7
(22) Date of filing: 16.05.2008
(51) Int. Cl.: C07C 213/08, C07C 215/64, C07B 41/10

(54) **Preparation of O-desmethylvenlafaxine salts**

(71) Applicant: KRKA, 8501 Novo mesto (SI)
(72) Inventor: Zupancic, Silvo, 8000 Novo mesto (SI); Vrecer, Frank, 8351 Straza pri Novem mestu (SI); Skrabanja, Vida, 8000 Novo mesto (SI); Hvalec, Miran, 8000 Novo Mesto (SI); Smrkolj, Matej, 1420 Trbovlje (SI); German, Tamara, 8000 Novo Mesto (SI)
(74) Representative: UEXKÜLL & STOLBERG

(57) **Abstract**

The present invention relates to a process for the preparation of a salt of O-desmethylvenlafaxine comprising
a) dissolving or suspending O-desmethylvenlafaxine in the form of its base in a liquid medium selected from the group of solvents and suspending agents;
b) contacting O-desmethylvenlafaxine with a pharmaceutically acceptable acid to form a pharmaceutically acceptable salt of O-desmethylvenlafaxine wherein the pharmaceutically acceptable acid is selected from the group consisting of HCl, HBr, H₂SO₄, H₃PO₄ acetic acid, adipic acid, galactaric acid, D-gluconic acid, glutamic acid, glutaric acid, glycolic acid, hippuric acid, lactic acid, oxalic acid, methanesulphonic acid, ethanesulphonic acid, p-toluenesulphonic acid, camphorsulphonic acid, benzenesulphonic acid, malonic acid, L-ascorbic acid, naphtalene-2-sulfonic acid and benzoic acid; and
c) separating the pharmaceutically acceptable salt of O-desmethylvenlafaxine from the liquid medium to recover the salt,

and to pharmaceutical formulations containing these salts.

## Description

The present invention provides novel processes for the preparation of pharmaceutically acceptable salts of O-desmethylvenlafaxine and pharmaceutical formulations containing the same.

O-desmethylvenlafaxine (hereinafter also "desvenlafaxine" or "ODV") is a major metabolite of venlafaxine and has been shown to inhibit norepinephrine and serotonin uptake. It is chemically named 1-[2-(dimethylamina)-1-(4-phenol)ethyl]cyclohexanol. Desvenlafaxine and methods for its preparation are described, e.g. in WO-A-00/59851 and WO-A-03/048104 (free-base), EP-A-0 112 669 (free-base and fumarate salt), WO-A-00/76955 (fumarate salts), WO-A-02/064543 (succinate salt) an WO-A-03/103603 (formate salt). However, there is still a need to provide alternative processes for the preparation of these and other pharmaceutically acceptable salts of desvenlafaxine, which processes are simple to conduct and which result in salts showing good solubility in solvents and allow one to obtain the salts in high yields.

The above problem is solved by a process as defined in claim 1. Preferred embodiments of the present invention are disclosed in the dependent claims.

The term "pharmaceutically acceptable salt" as used herein, also comprises hydrate forms, polymorphic forms, solvates of the salts where applicable. In cases where an acid is used with more than one acid moiety, i.e. a polycarboxylic acid such as a dicarboxylic acid or a tricarboxylic acid, the salt can be a full, hemi-salt or other intermediate salt where not all of the acid moieties are used for salt formation, as is e.g. the case in a 1 : 1 molar ratio salt of ODV with a tricarboxylic acid.

The term "solvent" as used herein means any organic or inorganic compound which is liquid at room temperature and suitable to partly or completely dissolve ODV either in the form of the free-base or a pharmaceutically acceptable salt.

The term "suspending agent" as used herein means any liquid medium suitable to suspend ODV or a pharmaceutically acceptable salt thereof, without all of ODV or the pharmaceutically acceptable salt thereof being dissolved.
**Figure 1** shows XRD data for the product of Example 2.
**Figure 2** shows micrographs of different magnifications (40x, 100x and 400x) for the product of Example 2.
**Figure 3** shows XRD data for the product of Example 3.
**Figure 4** shows XRD data for the product of Example 11.
**Figure 5** shows micrographs of different magnifications (40x, 100x and 400x) for the product of Example 11.
**Figure 6** shows XRD data for the product of Example 20.
**Figure 7** shows micrographs of different magnifications (40x, 100x and 400x) for the product of Example 20.

Specifically, according to a first aspect of the present invention provides a process for the preparation of a salt of O-desmethylvenlafaxine comprising
a) dissolving or suspending O-desmethylvenlafaxine in the form of its base in a liquid medium;
b) contacting O-desmethylvenlafaxine with a pharmaceutically acceptable acid to form a solution of a pharmaceutically acceptable salt of O-desmethylvenlafaxine wherein the pharmaceutically acceptable acid is selected from the group consisting of HCl, HBr, H₂SO₄, H₃PO₄, acetic acid, adipic acid, galactaric acid, D-gluconic acid, glutamic acid, glutaric acid, glycolic acid, hippuric acid, lactic acid, oxalic acid, methanesulphonic acid, ethanesulphonic acid, *p*-toluenesulphonic acid, camphorsulphonic acid, benzenesulphonic acid, malonic acid, L-ascorbic acid, naphtalene-2-sulfonic acid and benzoic acid, optionally filtering the solution; and
c) separating the pharmaceutically acceptable salt of O-desmethylvenlafaxine from the liquid medium to recover the salt.

The pharmaceutically acceptable acid of the present invention is thus selected from the group consisting of HCl, HBr, H₂SO₄, H₃PO₄, acetic acid, adipic acid, galactaric acid, D-gluconic acid, glutamic acid, glutaric acid, glycolic acid, hippuric acid, lactic acid, oxalic acid, methanesulphonic acid, ethanesulphonic acid, *p*-toluenesulphonic acid, camphorsulphonic acid, benzenesulphonic acid, malonic acid, L-ascorbic acid, naphtalene-2-sulfonic acid, benzoic acid. Preferably the acid is selected from the group consisting of HCl, HBr, H₂SO₄, H₃PO₄, D-gluconic acid, glycolic acid, hippuric acid, oxalic acid, methanesulphonic acid, ethanesulphonic acid, *p*-toluenesulphonic acid, benzenesulphonic acid, malonic acid and naphtalene-2-sulfonic acid.

According to the first aspect the liquid medium is a solvent selected from the group of alcohols such as methanol, ethanol, isopropanol, acetonitrile, tetrahydrofurane, acetone, methyl ethyl ketone, ethyl acetate, water or mixtures thereof.

In accordance with the invention, the solution may be formed after the addition of the acid or during or after heating to a temperature of up to the boiling point of the solvent used, in particular during or after heating under reflux conditions. After the solution of the pharmaceutically acceptable salt of ODV has been formed, the solvent can be removed by an evaporation process, a spray drying process or a lyophilization process.

Evaporation of the solvent is usually performed by distillation under reduced pressure. The temperature used during this process is usually between 10 and 100°C depending on the nature of the solvent used. The pressure of the process usually used is below normal pressure.

When spray-drying of the solvent is applied, desvenlafaxine and an acid are dissolved in an organic solvent in which the formed salt is well-soluble. Suitable examples include methanol, ethanol, isopropanol, acetonitrile, tetrahydrofurane, acetone and methyl ethyl ketone, water or mixtures thereof. The concentration of the desvenlafaxine salt can broadly vary and depends on the solubility of the salt in the chosen solvent. The solution is sprayed in a flow of hot gas in a suitable apparatus such as a Spray Dryer Büchi 190. By this process the solvent evaporates from the solution and solid amorphous desvenlafaxine salt precipitates. Gases applied for drying can be chosen from nitrogen, argon, carbon dioxide or air, or mixtures thereof. The temperature of the hot air can vary and usually depends on the temperature of the boiling point of the solvent used for the preparation of the solution of desvenlafaxine salt. After spray drying, a pure amorphous product is obtained which can optionally be additionally dried.

The lyophilization process consists of freezing, primary drying and secondary drying steps.

The freezing step consists of freezing the solution of desvenlafaxine salt. The solvents used for the preparation of solution can be selected from: Water, alcohols, ethers, DMF, DMA and similar solvents. Freezing is conducted in a freeze-drying apparatus. In this step, the solution is cooled below its eutectic point, i.e. the lowest temperature at which the solid and liquid phase of the material can coexist. This ensures that sublimation rather than melting will occur in the following steps. In the primary drying step, the pressure is lowered (below 10 mbar) to start the process of the sublimation of solvent. This process usually takes from 10 to 70 hours. The secondary drying step aims to sublimate the solvent molecules that are adsorbed during the freezing step, since the mobile solvent molecules were already sublimated in the primary drying phase. In this step, the temperature is raised higher than in the primary drying step, and can even be above 0 °C, to break any physico-chemical interactions that have formed between the water or solvent molecules and the frozen material. Usually the pressure is also lowered in this stage to encourage sublimation (typically in the range of microbars, or fractions of a pascal). After the freeze drying process is complete, the vacuum is usually broken with an inert gas, such as nitrogen, before the material is sealed.

Another option for separating the pharmaceutically acceptable salt of O-desmethylvenlafaxine from the liquid medium to recover the salt may be also performed by using supercritical fluid technology under supercritical conditions. The supercritical fluid is removed by depressuring it and allowing it to evaporate.

After separating the solvent, the salt is optionally suspended in organic materials, in particular those selected from the group consisting of ethers such as diethyl ether, diisopropyl ether *tert*-butyl methyl ether, petrol ether, THF and dioxane; esters such ethyl acetate, isopropyl acetate, propyl acetate, butyl acetate, isobutyl acetate, and *tert*-butyl acetate, suitable hydrocarbons such as hexane, heptane, cyclohexane, methylcyclohexane, toluene, xylene; alcohols such as ethanol, isopropanol, n-propanol, isobutanol, *tert*-butanol, n-butanol; and mixtures thereof, followed by filtering the suspension or optionally evaporating the solvent to dry the mixture. At the end of the preparation process as described above, the pharmaceutically acceptable ODV salt is isolated by collecting and drying.

Optionally the process of adding and evaporating suspending agent, filtering the residual suspension, drying and isolating the desvenlafaxine salt is repeated, as desired.

According to a second aspect of the present invention, the ODV salt is prepared without forming a solution. In this embodiment, the process comprises
a) suspending the base in a suspending agent at room temperature;
b) adding the pharmaceutically acceptable acid as defined above with regard to the first aspect, optionally heating of the suspension at a temperature of up to the boiling point of the suspending agent and for a period of 0 to 24 h and subsequently cooling the suspension if necessary; and
c) separating the salt from the suspending agent to recover the salt.

For example, the salt is separated from the liquid medium by filtering at a temperature of up to the boiling point of the solvent or by filtering after cooling to room temperature.

At the end of the preparation process as described above, the pharmaceutically acceptable ODV salt is isolated by collecting and drying.

### Dosage Forms

Desvenlafaxine in form of salts as described hereinabove can be incorporated into prolonged release solid dosage forms where the drug is released from the dosage form over prolonged time. Release over prolonged time means that 10-40% of drug dose is released from the dosage form (USP apparatus 2, water at 37°C) in 2 hours, 40-80% in 8 hours and more than 80% of drug dose in 16 hours. According to one aspect of the invention, prolonged release solid dosage form can be a multiparticulate dosage form or, according to another aspect, a single particulate dosage form.

In case of multiparticulate dosage forms the single dose of desvenlafaxine in the form of salts containing 10 to 200 mg, preferably 20 to 150 and most preferably 25 to 125 mg of drug, based on the free base, is split into a plurality of particles, wherein the particles are represented by pellets, micro tablets with a diameter of less than 4 mm, capsules or granules, and wherein a plurality of particles means at least 2 and up to several hundreds of particles.

Particles can be film-coated or uncoated, film-coated ones being preferred. Coated particles can be prepared by coating the core which contains desvenlafaxine in the form of free base or salts thereof formed with pharmaceutically acceptable acids selected from the group consisting of HCl, HBr, H₂SO₄, H₃PO₄, acetic acid, adipic acid, galactaric acid, D-gluconic acid, glutamic acid, glutaric acid, glycolic acid, hippuric acid, lactic acid, oxalic acid, methanesulphonic acid, ethanesulphonic acid, *p*-toluenesulphonic acid, camphorsulphonic acid, benzenesulphonic acid, malonic acid, L-ascorbic acid, naphtalene-2-sulfonic acid and benzoic acid with at least one film coating modulating the drug release kinetics in such a way that it is prolonged for at least 8 hours, preferably for at least 10 hours and most preferably for more than 12 hours.

A core containing desvenlafaxine can be present in the form of pellets with an average diameter of 0.25 to 1.5 mm, preferably 0.4 to 1.5 mm, most preferably 0.6 to 1.2 mm, tablets having a shape appropriate for further coating or granules. Drug containing cores in the form of pellets can be in the form of matrix pellets obtained by extrusion and spheronization or by direct pelletization in a high shear mixer or rotor pelletizator or in the form of drug layered pellets where the drug together with other excipients is layered onto a neutral core such as neutral pellets made of sucrose and starch or microcrystalline cellulose.

Tablet cores can be manufactured by direct compression or via granulation using either wet or dry processes, according to state of the art processes.

Film coating modulating drug release is based on substances with low solubility in water, which can form a film around drug containing core which retards the release rate of drug from the core. Low solubility substances can be selected from polymers such as ethyl cellulose, polyvinylacetate, ammonio methacrylate copolymers (Eudragit RS and/or Eudragit RL), methacrylic ester copolymers (Eudragit NE), methylmetacrylate ethylmetacrylate copolymer (Kollicoat EMM). Film coating can optionally be composed of further pharmaceutically acceptable excipients selected from plasticizers, pore formers, antitacking agents, glidants (colloidal silica - Aerosil), pigments and colorants.

Plasticizers can be selected from polyethylene glycols such as PEG 6000, triacetine, triethylcitrate, dibutylsebacate, etc.

Pore formers can be selected from the group of small molecules with a molecular weight (Mw) of less than 1,000 with a solubility of lg in less than 20 ml of water, preferably in less than 15 ml and most preferably in less than 10 ml of water such as lactose, mannitol, sorbitol, urea, sucrose, trehalose, and/or from the group of water soluble polymers such as povidone with K value below 50, copovidone, hydroxypropylmethyl cellulose having viscosity of 2% solution in water at room temperature below 100 mPas, hydroxypropyl cellulose, methylcellulose, hydroxyethylcellulose, polyvinyl alcohol, polyethylenglycols with a MW below 10,000.

In a special embodiment of the invention, desvenlafaxine salt is formed *in situ* by dissolving or suspending the desvenlafaxine free base and the organic or anorganic acid selected from the group consisting of HCl, HBr, H₂SO₄, H₃PO₄, acetic acid, adipic acid, galactaric acid, D-gluconic acid, glutamic acid, glutaric acid, glycolic acid, hippuric, lactic acid, oxalic acid, methanesulphonic acid, ethanesulphonic acid, *p*-toluenesulphonic acid, camphorsulphonic acid, benzenesulphonic acid, malonic acid, L-ascorbic acid, naphtalene-2-sulfonic acid and benzoic acid in purified water or an organic medium such as alcohols such as methanol, ethanol, isopropanol; acetonitrile; tetrahydrofurane; acetone and methyl ethyl ketone; ethyl acetate; water or mixtures thereof, wherein the molar ratio of desvenlafaxine and acid is 1:1; 2:1 and/or 3:1. Further excipients such as binders, fillers, pH modifiers, anti-tacking agents can optionally be dispersed in the obtained solution. The obtained dispersion (solution/suspension) can be sprayed onto inert seeds such as neutral pellets made of sucrose and starch, microcrystalline cellulose or lactose or granulated by the processes known from the state of the art. The obtained layered pellets are dried and sieved and coated in same equipment with at least one film coating which decreases the release rate of drug from pellets. Optionally a separating coating can be applied onto pellets coated with drug layer, thereby preventing direct contact of the drug layer with the controlled release coating.

In order to achieve an optimal yield of layering with drug suspended in solvent, particle size of desvenlafaxine salt is important. In particular, a desvenlafaxine salt particles with an average particle size of less than 50pm, preferably less than 30µm and most preferably less than 20µm are used. Images of particles were taken on a Microscope Olympus BX 50 equipped with Olympus camera DP70. Sample was prepared as suspension in paraffin oil.

In another embodiment of the invention desvenlafaxine is incorporated into single unit prolonged release dosage form such as prolonged release matrix tablets and/or prolonged release matrix coated tablets.

Prolonged release matrix tablet comprise desvenlafaxine in the form of free base or pharmaceutically acceptable salts thereof having melting point higher than 70°C, prolonged release polymer, diluent and optionally other excipients such as disintegrator, binder, lubricant and/or glidant. Prolonged release matrix tablets can be produced by the state of the art processes and equipment, such as direct compression, compression of pregranulated compositions, where dry granulation such as roller compaction or slugging, wet granulation or melt granulation are used.

Prolonged release polymers can be selected from hydrophilic polymers such as cellulose ethers such as high viscosity grades of hydroxypropylmethyl cellulose having a viscosity measured according to USP (2% solution in water at 20°C) of more than 2,000 cP and hydroxypropyl cellulose having a viscosity (1% solution in water at 20°C, Brookfield viscosimeter) of more than 1,000 mPas, methyl cellulose, polyethylene oxides having molecular weight of more than 500,000; preferably of more than 1,000,000; carbomers such as Carbopol^{®}, methacrylic acid copolymers such as Eudragit NE^{®} 30 D, caragenans, xanthane, locust bean gum or hydrophobic polymers such as polyvinyl acetate, ethycellulose, ammonio methacrylate copolymers or mixtures thereof.

Diluents can be selected from water soluble diluents such as lactose, mannitol, or other water soluble carbohydrates, or water unsoluble diluents such as dicalcium phosphate (in hydrated or unhydrous state), crystalline cellulose (e.g. Avicel types), silicified cellulose (Prosolv), starch derivatives such as corn starch, pregelatinized starch or mixtures thereof.

Binders can be selected from polymers such as water soluble cellulose ethers, povidone, copovidone, polyvinyl alcohol and or substances having low melting point, where the melting point of binder is below 70°C, preferably below 60°C, such as polyethylene glycols, esters of fatty acids such as glycerol monostearate or mixtures thereof.

Disintegrators can be' selected from microcrystalline cellulose, starch and its derivatives such as pregelatinized starch or sodium starch glycolate, crospovidone, crosscarmelose sodium, low substituted hydroxypropyl cellulose, calcium carboxymethylcellulose, crosslinked carboxymethyl cellulose, polacrylin potassium, alginic acid, sodium alginate or mixtures thereof.

Glidants are selected from colloidal silicon dioxide (Aerosil), talc, magnesium trisilicate or mixtures thereof.

Lubricants are selected from metal salts of fatty acids such as magnesium, aluminium, calcium or zinc stearates, hydrogenated castor oil, talc, sodium stearyl fumarate, stearic acid or mixtures thereof.

In a special embodiment of the invention, desvenlafaxine salt is formed *in situ* by dissolving or suspending the desvenlafaxine free base and the organic or anorganic acid selected from the group consisting of HCl, HBr, H₂SO₄, H₃PO₄, acetic acid, adipic acid, galactaric acid, D-gluconic acid, glutamic acid, glutaric acid, glycolic acid, hippuric, lactic acid, oxalic acid, methanesulphonic acid, ethanesulphonic acid, *p*-toluenesulphonic acid, camphorsulphonic acid, benzenesulphonic acid, malonic acid, L-ascorbic acid, naphtalene-2-sulfonic acid and benzoic acid in purified water or an organic medium such as alcohols such as methanol, ethanol, isopropanol; acetonitrile; tetrahydrofurane; acetone and methyl ethyl ketone; ethyl acetate; water or mixtures thereof, wherein the molar ratio of desvenlafaxine and acid is 1:1; 2:1 and/or 3:1. Further excipients such as binders, fillers, pH modifiers, antitacking agents can optionally be dispersed in the obtained solution. This dispersion (solution/suspension) can be used as granulating fluid and sprayed onto an excipient or mixture of excipients, containing filler and optionally other excipients such as prolonged release polymers, binders, pH modifiers, water permeation enhancers, stabilizers by conventional pharmaceutical techniques for granulation. The obtained granulate is further mixed with at least one excipients selected from diluents, prolonged release polymers, glidants, lubricants and pressed into matrix tablets.

Prolonged release coated matrix tablets can be produced by coating the prolonged release matrix tablet with film coatings which are in one aspect of invention soluble in water and are applied onto the matrix tablet in order to improve the physical appearance of the tablet such as colour, taste or smell, ease the swallowing of the tablet, improve physical and chemical stability of the ingredients in the tablet, especially the desvenlafaxine and its salts by diminishing the permeation of water vapour and/or oxygen into the core resulting in chemical degradation of ingredients specially active drug substance. Water soluble film coatings are applied by the processes and equipment known from the state of the art, such as coating in perforated coating pansor Wurster fluid bed equipment, by spraying the dispersion of an appropriate water soluble polymer and other inactive ingredients such as pigments, plasticizers, glidants, antitacking agents in water, water miscible organic solvents, such as alcohols or ketones or mixtures thereof. Appropriate polymers can be selected from soluble viscosity types of hydropropylmethyl cellulose, methyl' cellulose, polyvinyl alcohol, aminoalkyl methacrylate copolymers (Eudragit EPO), sodium carboxymethylcellulose or the like. Pigments can be selected from metal oxides such iron oxides, titanium oxide.

In another embodiment of the invention, the film coating can be applied onto the matrix tablet in order to achieve lag time before the start of dissolution of active drug substance from the core or to further diminish the release rate of active drug substance from the dosage form. Polymers for achieving lag time in dissolution can be selected from those used for gastro resistant coating such as hydropropylmethyl cellulose acetate succinate, methacrylic acid ethylacrylate copolymer, hydropropylmethyl cellulose acetate phthalate.

Drug release from prolonged release rate matrix tablets can be further decreased by applying a prolonged release film coating based on water insoluble polymers such as ethyl cellulose, polyvinylacetate, ammonio methacrylate copolymers (Eudragit RS and/or Eudragit RL), methacrylic ester copolymers (Eudragit NE), methylmetacrylate ethylmetacrylate copolymer (Kollicoat EMM). Further excipients selected from plasticizers, antitacking agents, pore formers, glidants can be used in the coating.

Low gas permeable primary packaging materials such as aluminium or polychloro-3-fluoroethylene homopolymer/PVC laminate can be used with the thickness in the range 10 to 40 µm in case of Al/Al blisters and 10 to 110 µm in case of Al-polychloro-3-fluoroethylene homopolymer/PVC laminate blisters. Optionally, dosage forms containing desvenlafaxine or its pharmaceutically acceptable salt can be packed into primary packaging with desiccant. Desiccant can be placed inside the packaging unit together with dosage units such as tablets and/or in the closure system or can be incorporated into the walls of the primary packaging unit.

To avoid the potential oxidative degradation of incorporated active and inactive ingredients susceptible to oxidative degradation, the final dosage form can be packed in primary packaging under inert atmosphere such as for example nitrogen, argon or xenon resulting in decreased concentration of oxygen in the atmosphere surrounding the dosage form in primary packaging such as for example blisters, strips, plastic or glass containers. Decreased concentration of oxygen means, that the concentration of residual oxygen in the atmosphere surrounding the individual dosage form such as for example tablet or capsule is below 10 vol/vol%, preferably below 7.5 vol/vol%, more preferably below 5 vol/vol% and most preferably below 2.5 vol/vol%.

### Examples

The following examples illustrate the present invention. Examples 1 to 22 illustrate the processes for preparing the pharmaceutically acceptable salts.

Melting points were taken on a Koffler melting point apparatus and IR spectra were taken on a Paragon 100 Perkin-Elmer FT-IR spectrometer.

X-ray powder diffraction patterns were obtained by a Phillips PW3040/60 X'Pert PRO diffractometer using CUK_{α} radiation of 1,541874 Å.

FT-IR analysis was performed on FT-IR System SPECTRUM GX Perkin Elmer [4000-400] cm⁻¹. Resolution 4 cm⁻¹, KBr tbl.

Examples 23 to 32 illustrate the preparation of formulations containing said salts.

### Example 1: Desvenlafaxine hydrochloride (36)

1.05 g (4 mmol) of desvenlafaxine was suspended in 15 ml of methanol and 1.3 ml of 3M HCl was added. The suspension was heated to reflux temperature and heated at this temperature for 1 h. The clear solution was obtained and this solution was cooled to RT and the solvent was evaporated. 1.0 g of desvenlafaxine hydrochloride was isolated.
IR: 2934, 1616, 1517, 1264, 1150, 839, 668

This product vas suspended in diethyl ether for 1h and the suspension was filtered and the product was dried. 0.7 g of desvenlafaxine hydrochloride was isolated.
Mp = 90 - 105°C
IR: 2934, 1616, 1518, 1449, 1270, 1150, 952, 849

### Example 2: Desvenlafaxine hydrochloride (61)

0.53 g (2 mmol) of desvenlafaxine was suspended in 7.5 ml of water and 0.65 ml of 3M HCl was added. After the addition of the acid the solution was formed. This solution was filtered and filtrate was evaporated. To the residue isopropanol was added and isopropanol was evaporated to isolate solid desvenlafaxine hydrochloride (0.55 g).
IR: 2935, 1614, 1517, 1448, 1224, 951, 840, 591

This product was suspended in diethyl ether for 1h and the suspension was filtered and the product was dried.
IR: 2938, 1614, 1517, 1448, 1269, 1149, 951, 846

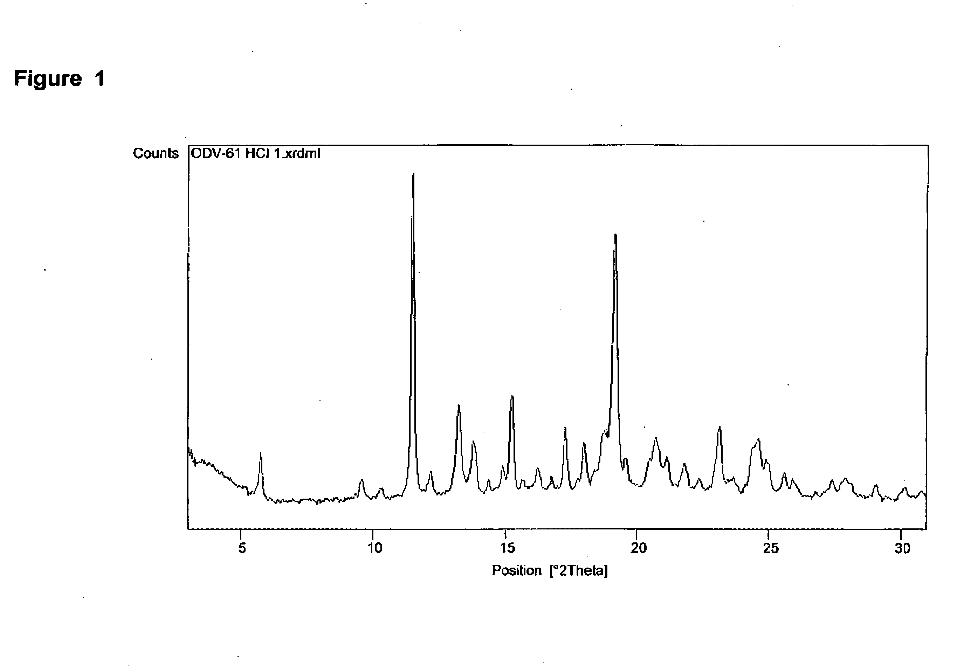

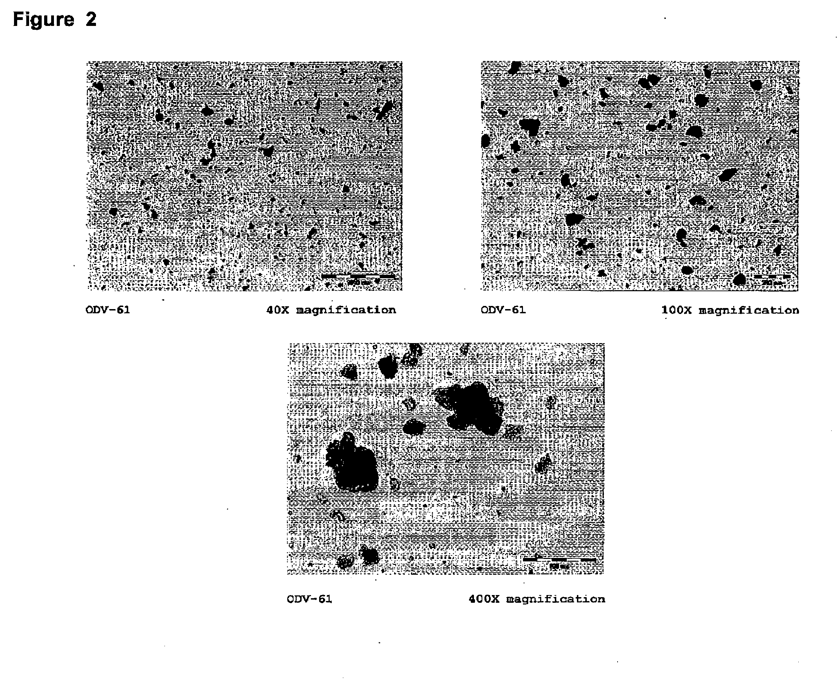

XRD data for this product are shown in **Fig. 1****.** **Fig. 2** shows micrographs of different magnifications (40x, 100x and 400x). The following table lists the peak intensities of the peaks shown in the diffractogramm of **Fig. 1****:**

| No. | Pos. [°2Th.] | d-spacing [A] | Rel.Int. [%] |
|---|---|---|---|
| 1 | 11.5 | 7.67 | 100 |
| 2 | 13.3 | 6.67 | 33 |
| 3 | 15.3 | 5.80 | 36 |
| 4 | 17.3 | 5.12 | 27 |
| 5 | 19.2 | 4.62 | 81 |
| 6 | 23.1 | 3.84 | 28 |
| 7 | 24.5 | 3.63 | 23 |

### Example 3: Desvenlafaxine hydrochloride (64)

2.05 g (8 mmol) of desvenlafaxine was suspended in 30 ml of water and 2.67 ml of 3M HCl was added. After the addition of the acid the solution was formed. This solution was filtered and the filtrate was spray dried to isolate solid desvenlafaxine hydrochloride (1.5 g).
Mp = 92-100 °C
IR: 2936, 1614, 1517, 1265, 1178, 1150, 964, 840

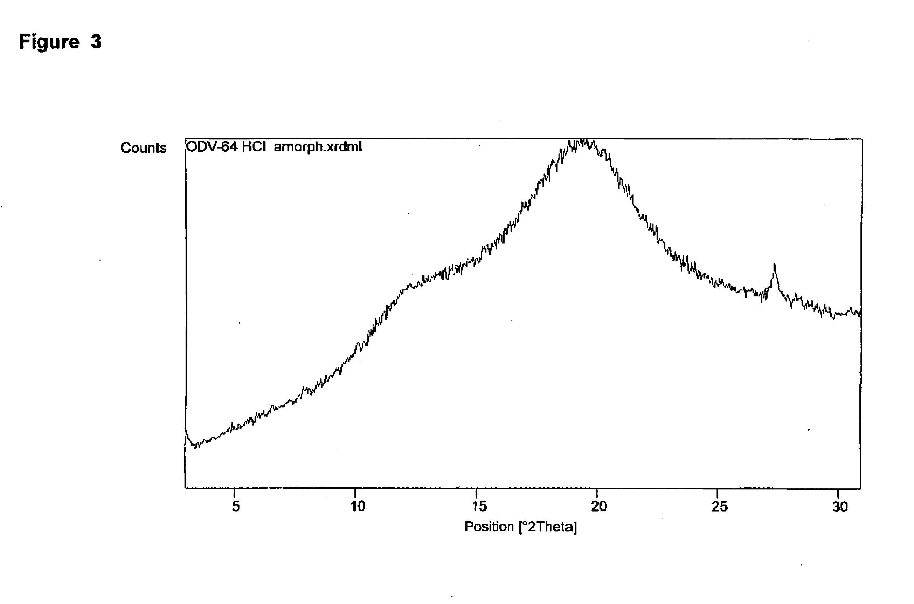

XRD data for this product are shown in Fig. 3.

### Example 4: Desvenlafaxine sulphate (52)

1.05 g (4 mmol) of desvenlafaxine was suspended in 15 ml of water and 4 ml of 1 M H₂SO₄ was added. After the addition of the acid the solution was formed. This solution was filtered and the filtrate was evaporated. To the residue isopropanol was added and evaporated, the residue was suspended in diethyl ether, filtered and dried to isolate solid desvenlafaxine sulphate (0.86 g).
IR: 2937, 1615, 1517, 1448, 1224, 1055, 951, 840, 592

### Example 5: Desvenlafaxine hemisulphate (68)

2.11 g (8 mmol) of desvenlafaxine was suspended in 30 ml of water and 4 ml of 1 M H₂SO₄ was added. After the addition of the acid the solution was formed. This solution was filtered and lyophilized. After this process was completed 2.32 g of desvenlafaxine hemisulphate hydrate was isolated.
Mp = 113-133 °C
KF water content: 7.245
IR: 2935, 1614, 1517, 1448, 1267, 1125, 965, 840, 619

### Example 6: Desvenlafaxine hemisulphate (89)

0.685 g (2.3 mmol) of desvenlafaxine was suspended in 10 ml of 1,4-dioxan and 1.3 ml (1.3 mmol) of 1M H₂SO₄ was added. After the addition of acid the solution was formed at elevated temperature. This solution was filtered and evaporated. To the solid residue, isopropyl acetate was added and evaporated again. To the solid residue 5 ml of isopropyl acetate was added, stirred and the product was separated. 0.81 g of desvenlafaxine hemisulphate was isolated.
Mp = 124-145 °C
KF water content: 3.29%
IR: 2929, 1616, 1517, 1457, 1255, 1120, 966, 872, 840, 618

### Example 7: Desvenlafaxine phosphate (70)

2.11 g (8 mmol) of desvenlafaxine was suspended in 30 ml of water and 0.54 ml (8 mmol) of 85% H₃PO₄ was added. After the addition of the acid the solution was formed. This solution was filtered and lyophilized. After this process was completed 2.65 g of desvenlafaxine phosphate hydrate was isolated.
Mp = 105-120 °C
KF water content: 6.72%
IR: 2938, 1614, 1517, 1448, 1265, 1068, 951, 840, 546

### Example 8: Desvenlafaxine phosphate (78)

2.11 g (8 mmol) of desvenlafaxine was suspended in 30 ml of water and 0.54 ml (8 mmol) of 85% H₃PO₄ was added. After the addition of the acid the solution was formed. This solution was filtered and lyophilized. After this process was completed 2.65 g of desvenlafaxine phosphate hydrate was isolated.
Mp = 98-119 °C
KF water content: 6.18%
IR: 2937, 1614, 1517, 1448, 1265, 1068, 951, 840, 526

### Example 9: Desvenlafaxine phosphate (86)

1.05 g (4 mmol) of desvenlafaxine was suspended in 15 ml of water and 0.27 ml (4 mmol) of 85% H₃PO₄ was added. After the addition of acid the solution was formed. This solution was filtered and filtrate was evaporated to the solid residue. To the residue isopropanol was added and the solvent is evaporated again. The residue is then triturated with isopropanol and the precipitate is filtered and dried (0.78 g).
Mp = 110-130 °C
KF water content: 5.47%
IR: 2935, 1614, 1517, 1465, 1267, 1068, 951, 840, 525

### Example 10: Desvenlafaxine oxalate (54)

1.05 g (4 mmol) of desvenlafaxine was suspended in 15 ml of methanol and 0.18 ml (2 mmol) of oxalic acid was added. The suspension was heated at reflux temperature for 1 h. The solution was filtered, cooled to RT and the solvent was evaporated. The residue was suspended in 15 ml of diethyl ether. The product was filtered, dried and 0.71 g of desvenlafaxine hemioxalate was isolated.
Mp = 117- 130°C.
IR: 2934, 1616, 1517, 1457, 1270, 1152, 968, 840, 764

### Example 11: Desvenlafaxine oxalate (53)

1.05 g (4 mmol) of desvenlafaxine was suspended in 15 ml of methanol and 0.36 ml (4 mmol) of oxalic acid was added. The suspension was heated at reflux temperature for 1 h. The solution was filtered, cooled to RT and the solvent was evaporated. The residue was suspended in 15 ml of diethyl ether. The product was filtered, dried and 0.70 g of desvenlafaxine oxalate was isolated.
Mp = 66 - 80°C.
IR: 2936, 1724, 1615, 1517, 1450, 1235, 951, 840, 712

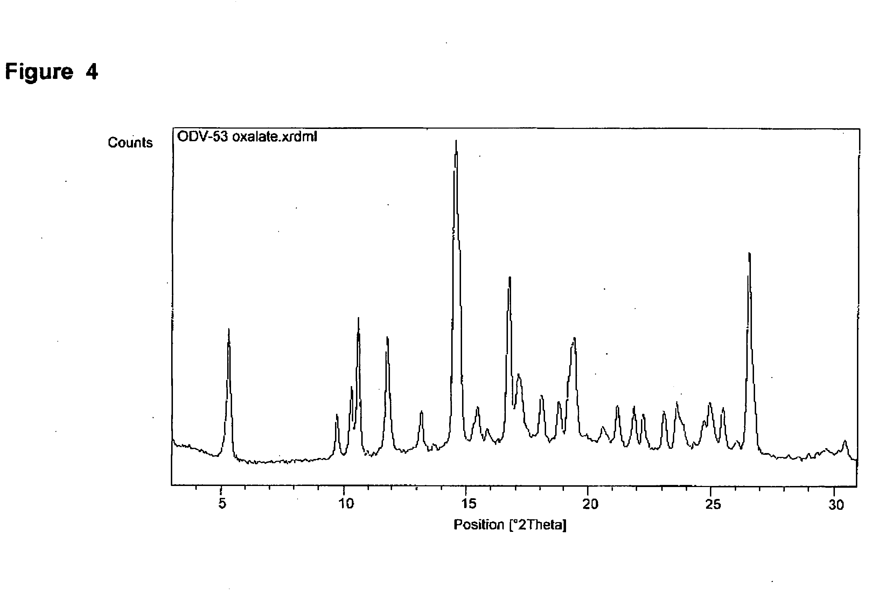

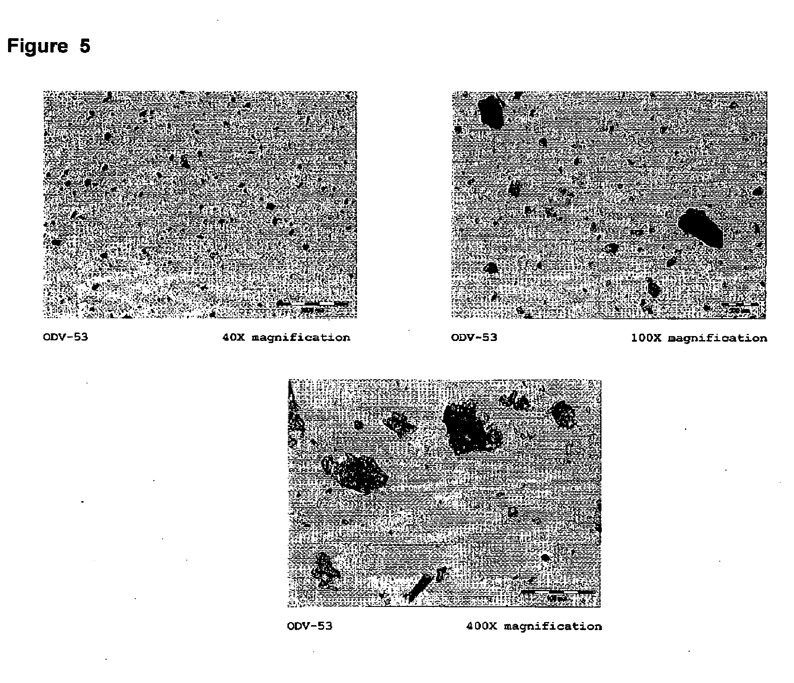

XRD data for this product are shown in **Fig. 4****.** **Fig. 5** shows micrographs of different magnifications (40x, 100x and 400x). The following table lists the peak intensities of the peaks shown in the diffractogramm of **Fig. 4****:**

| No. | Pos. [°2Th.] | d-spacing [A] | Rel. Int. [%] |
|---|---|---|---|
| 1 | 5.3 | 16.64 | 46 |
| 2 | 10.6 | 8.35 | 50 |
| 3 | 11.8 | 7.51 | 44 |
| 4 | 14,5 | 6.09 | 100 |
| 5 | 16,7 | 5.30 | 62 |
| 6 | 19.5 | 4.56 | 43 |
| 7 | 26.5 | 3.36 | 70 |

### Example 12: Desvenlafaxine methanesulphonate (50)

1.05 g (4 mmol) of desvenlafaxine was suspended in 5 ml of mixture of methanol / water and 0.77 ml (4 mmol) of methanesulphonic acid was added. After the addition of acid the solution was formed. This solution was filtered and filtrate was evaporated. The residue was suspended in diethyl ether, filtered and dried to isolate solid desvenlafaxine methanesulphonate (0.96 g).
Mp = 103-114°C.
IR: 2938, 1633, 1616, 1518, 1449, 1395, 1233, 966, 829

### Example 13: Desvenlafaxine benzenesulphonate (93)

1.05 g (4 mmol) of desvenlafaxine was suspended in 15 ml of water and 0.67 ml (4 mmol) of benzenesulphonic acid was added. After the addition of acid the solution was formed. This solution was filtered and filtrate was evaporated. The residue was further processed by adding and evaporating isopropanol. After that, to the residue 5 ml of isopropanol was added and the formed suspension stirred for 1h at room temperature. Then the suspension was filtered and dried to isolate solid desvenlafaxine benzenesulphonate (0.56 g).

### Example 14: Desvenlafaxine p-toluenesulphonate (97)

1.05 g (4 mmol) of desvenlafaxine was suspended in 15 ml of water and 0.76 ml (4 mmol) of *p*-toluenesulphonic acid was added. After the addition of acid the solution was formed. This solution was filtered and filtrate was evaporated. The residue was further processed by adding and evaporating isopropanol. After that, to the residue 5 ml of isopropanol was added and the formed suspension was stirred for 1h at room temperature. Then the suspension was filtered and dried to isolate solid desvenlafaxine *p*-toluenesulphonate (0.69 g).
IR: 2936, 1723, 1616, 1517, 1450, 1373, 1123, 1034, 1011, 960, 840, 816, 683, 568

### Example 15: Desvenlafaxine ethanesulphonate (88)

1.05 g (4 mmol) of desvenlafaxine was suspended in 15 ml of water and 0.47 ml (4 mmol) of ethanesulphonic acid was added. After the addition of acid the solution was formed. This solution was filtered and filtrate was evaporated. The residue was dried by addition and evaporation of isopropanol. After that, to the residue 5 ml of diethyl ether was added and the formed suspension was stirred for 1h at room temperature. Then the suspension was filtered and dried to isolate solid desvenlafaxine ethanesulphonate (0.69 g).
IR: 2936, 1616, 1595, 1517, 1451, 1243, 1136, 1040, 967, 960, 841, 744, 622, 576

### Example 16: Desvenlafaxine D-gluconate (96)

1.05 g (4 mmol) of desvenlafaxine was suspended in 15 ml of water and 1.57 ml (4 mmol) of 50% D-gluconic acid was added. After the addition of acid the solution was formed. This solution was filtered and filtrate was evaporated. The residue was dried by addition and evaporation of isopropanol. 1.6 g of desvenlafaxine D-gluconate as semi solid was isolated.
IR: 2931, 1778, 1603, 1516, 1266, 1200, 952, 840

### Example 17: Desvenlafaxine glycolate (92)

1.05 g (4 mmol) of desvenlafaxine was suspended in 15 ml of water and 0.30 g (4 mmol) of glycolic acid was added. After the addition of acid the solution was formed. This solution was filtered and filtrate was evaporated. The residue was further processed by adding and evaporating isopropanol. 1.2 g of desvenlafaxine glycolate as semi solid was isolated.
IR: 2935, 1600, 1516, 1270, 1135, 1109, 952, 842, 746, 700

### Example 18: Desvenlafaxine malonate (31)

In 15 ml of methanol 1.05 g (4 mmol) of desvenlafaxine was suspended and 0.42 ml (4 mmol) malonic acid was added. The suspension was heated at reflux temperature for 1 h. The solution was filtered, cooled to RT and the solvent was evaporated. The residue was suspended in 20 ml of *tert-*butylmethyl ether. The product was filtered, dried and 0.75 g of desvenlafaxine malonate was isolated.
IR: 2935, 1617, 1616, 1516, 1455, 1265, 1178, 1042, 956, 839, 758

The following examples illustrate the processes of the present invention wherein a suspension is used to prepare the salts.

### Example 19: Desvenlafaxine hippurate (11)

In 15 ml of methanol 1.05 g (4 mmol) of desvenlafaxine was suspended and 0.72 g (4 mmol) hippuric acid was added. The suspension was heated at reflux temperature for 1 h. Then the suspension is cooled and filtered. Title product was dried (0.91 g).
Mp = 135-141°C
IR: 2939, 1637, 1619, 1518, 1276, 842, 722

### Example 20: Desvenlafaxine ethanesulphonate (12)

In 15 ml of methanol 1.05 g (4 mmol) of desvenlafaxine was suspended and 0.47 ml (4 mmol) ethanesulphonic acid was added. The suspension was heated at reflux temperature for 1 h. Then the suspension is cooled and filtered. Title product was dried and 0.81 g of desvenlafaxine ethanesulphonate was isolated.
Mp = 100-109 °C.
IR: 937, 1623, 1519, 1418, 1248, 1264, 1136, 1064, 952, 757, 586

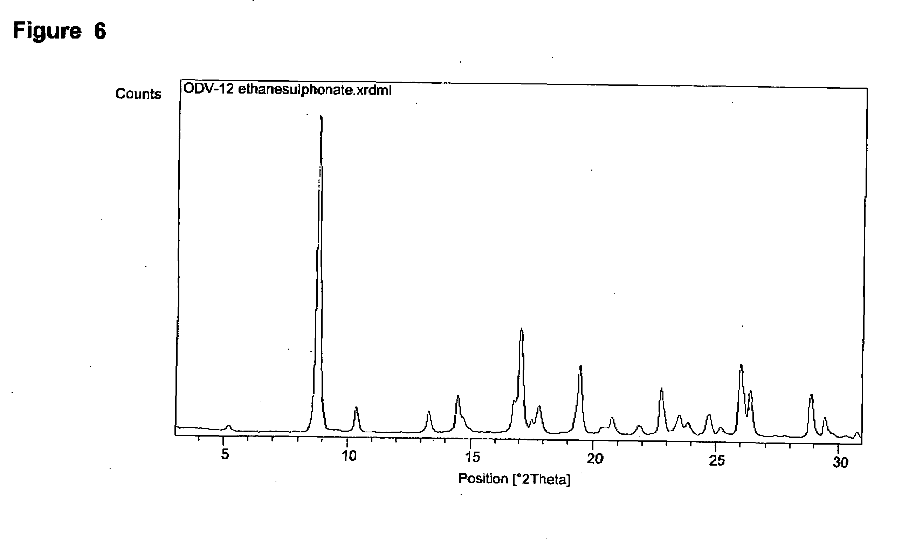

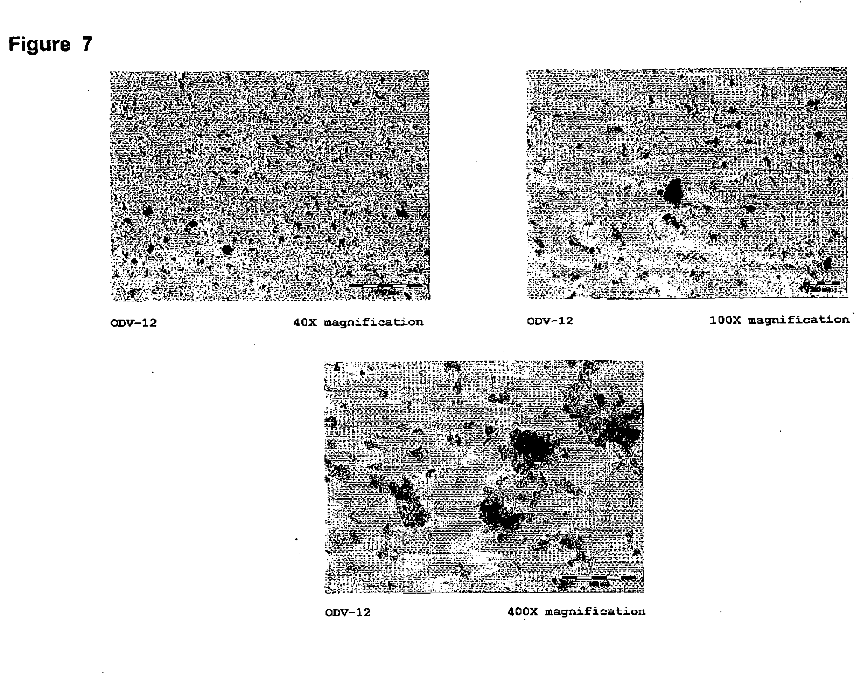

XRD data for this product are shown in **Fig. 6****.** **Fig. 7** shows micrographs of different magnifications (40x, 100x and 400x). The following table lists the peak intensities of the peaks shown in the diffractogramm of **Fig. 6****:**

| No. | Pos. [°2Th.] | d-spacing [A] | Rel. Int. [%] |
|---|---|---|---|
| 1 | 8.8 | 10.09 | 100 |
| 2 | 14.5 | 6.12 | 14 |
| 3 | 17.1 | 5.20 | 34 |
| 4 | 19.5 | 4.56 | 23 |
| 5 | 22.8 | 3.91 | 16 |
| 6 | 26.0 | 3.43 | 24 |
| 7 | 28.8 | 3.10 | 15 |

### Example 21: Desvenlafaxine glycolate (10)

In 15 ml of methanol 1.05 g (4 mmol) of desvenlafaxine was suspended and 0.30 g (4 mmol) glycolic acid was added. The suspension was heated at reflux temperature for 1 h. Then the suspension is cooled and filtered. Title product was dried and 0.63 g of desvenlafaxine glycolate was isolated.
Mp = 223-228 °C
IR: 2939, 1618, 1518, 1447, 1277, 1148, 1098, 842

The following examples illustrate the preparation of pharmaceutical formulations.

### Example 22: Desvenlafaxine (100)

1.00 g of crude desvenlafaxine was suspended in 55 ml of methanol and heated to reflux temperature and the active ingredient was almost completely dissolved in the mixture. Hot solution was filtered and filtrate was stirred at room temperature for 2 h. The product was filtered, washed with fresh methanol and dried. 0.65 g of title product was isolated.
Mp = 191-220 °C
KF water content: 3.08%
IR: 2940, 1619, 1517, 1460, 1446, 1277, 1241, 1147, 1038, 964, 845

### Example 23: coated dosage form with in situ preparation of salt of ODV

### A) Drug layered pellets:

| | |
|---|---|
| Sugar spheres (0.6-0.71 mm) | 1.400.00 g |
| ODV | 210.72 g |
| Pharmacoat 603 | 50.00 g |
| Hydrochloric acid 3 M | 267.40 ml |
| Purified water | q.s. |

ODV is suspended in purified water and 3M HCl is added to the suspension while mixing. The obtained dispersion is left mixing until a clear solution is obtained. Pharmacoat 603 is dissolved in the obtained solution and sprayed onto sugar spheres using Wurster insert of GPCG-3. Product temperature is kept between 35 and 45°C. Pellets are dried in same equipment until loss on drying (Mettler halogen dryer at 85°C/20 min) is below 1.3%. Dried pellets are sieved through 1 mm sieve and used for further coating.

### B) pellets coated with a separating layer:

| | |
|---|---|
| pellets obtained in step 1A | 1,436.50 g |
| Pharmacoat 603 | 75.00 g |
| Talc | 25.00 g |
| Purified water | q.s. |

Talc is homogenously dispersed in part of water. The suspension is added to the solution of Pharmacoat 603 in purified water. The obtained dispersion is sprayed onto pellets obtained in step 1A. Pellets are dried and sieved as described in step 1A.

### C) pellets coated with pronged release coating

| | |
|---|---|
| Pellets obtained in step 1B | 1,400.00 g |
| Aquacoat ECD | 470.00 g |
| Dibutylsebacate | 35.00 g |
| Purifed water | 95.00 g |

Dibutylsebacate is slowly added to the Aquacoat while gentle mixing. Water is added to the obtained emulsion and left mixing for 15 minutes. The obtained dispersion is sprayed onto pellets obtained in step 1B in same equipment as used in previous steps. Product temperature is kept 32-35°C during the coating. After the coating pellets are dried and sieved. Sieved pellets,are cured 3 hours at 60°C in drying chamber.

Pellets are filled into capsules containing 100 mg ODV corresponding to 113.9 mg desvenlafaxine hydrochloride salt per capsule.

### Example 24: Film coated matrix tablets containing 100 mg of desvenlafaxine in form of sulphate salt

| Composition per tablet | |
|---|---|
| Desvenlafaxine sulphate | 137.3 mg |
| Methocel K 100M SR | 220.0 mg |
| Avicel pH 101 | 135.0 mg |
| Aerosil 200 | 2.7 mg |
| Magnesium stearate | 5.0 mg |

Desvenlafaxine sulphate is mixed with Methocel K 100M SR and Avicel. After that Areosil is admixed to the obtained mixture and finally magnesium stearate is homogenously admixed. The obtained powder mixture is compressed into 500 mg oblong tablets.

The obtained tablets were coated with coating (10% weight gain calculated on the weight of the core) with following compostion:

| | |
|---|---|
| Sodium carboxymethylcellulose | 80.0% |
| Maltodextrin | 10.0% |
| Dextrose | 6.5% |
| Titanium dioxide | 1.0% |
| Stearic acid | 2.0% |
| Iron oxide | 0.5% |

### Example 25: Film coated matrix tablets containing 100 mg of desvenlafaxine in form of malonate salt

| Composition per tablet | |
|---|---|
| Desvenlafaxine malonate | 139.6 mg |
| Methocel K 250M SR | 200.0 mg |
| Avicel pH 101 | 48.4 mg |
| Talc | 10.0 mg |
| Magnesium stearate | 2.0 mg |

Desvenlafaxine malonate is mixed with Methocel K 250M SR, ethylcellulose and Avicel. After that talc is admixed to the obtained mixture and finally magnesium stearate is homogenously admixed. The obtained powder mixture is compressed into 400 mg oblong tablets.

The obtained tablets were coated with Opadry AMB coating (10% weight gain calculated on the weight of the core).

### Example 26: Film coated matrix tablets containing 100 mg of desvenlafaxine in form of sulphate salt

| Composition per tablet | |
|---|---|
| Desvenlafaxine sulphate | 34.3% |
| Polyox WSR 301 | 40.0% |
| Avicel pH 101 | 24.5% |
| Aerosil 200 | 0.7% |
| Magnesium stearate | 0.5% |

Desvenlafaxine sulphate is mixed with Polyox WSR 301 (polyethyle oxide polymer) and Avicel. After that Areosil is admixed to the obtained mixture and finally magnesium stearate is homogenously admixed. The obtained powder mixture is compressed into 300 mg oblong tablets.

The obtained tablets were coated with coating (10% weight gain calculated on the weight of the core) with following compostion:

| | |
|---|---|
| Sodium carboxymethylcellulose | 80.0% |
| Maltodextrin | 10.0% |
| Dextrose | 6.5% |
| Titanium dioxide | 1.0% |
| Stearic acid | 2.0% |
| Iron oxide | 0.5% |

### Example 27: Film coated matrix tablets containing 75 mg of desvenlafaxine in form of sulphate salt

| Composition per tablet | |
|---|---|
| Desvenlafaxine sulphate | 34.3% |
| Polyox WSR 301 | 40.0% |
| Avicel pH 101 | 24.5% |
| Aerosil 200 | 0.7% |
| Magnesium stearate | 0.5% |

Desvenlafaxine sulphate is mixed with Polyox WSR 301 (polyethyle oxide polymer) and Avicel. After that Areosil is admixed to the obtained mixture and finally magnesium stearate is homogenously admixed. The obtained powder mixture is compressed into 300 mg oblong tablets.

The obtained tablets were coated with coating (10% weight gain calculated on the weight of the core) with following composition:

| | |
|---|---|
| Sodium carboxymethylcellulose | 80.0% |
| Maltodextrin | 10.0% |
| Dextrose | 6.5% |
| Titanium dioxide | 1.0% |
| Stearic acid | 2.0% |
| Iron oxide | 0.5% |

### Example 28: Film coated matrix tablets containing 75 mg of desvenlafaxine in form of sulphate salt

| Composition per tablet | |
|---|---|
| Desvenlafaxine sulphate | 34.3% |
| Polyox WSR N-1105 | 55.0% |
| Dicalcium phosphate dihydrate | 9.5% |
| Aerosil 200 | 0.7% |
| Magnesium stearate | 0.5% |

Desvenlafaxine sulphate is mixed with Polyox WSR N-1105 (polyethyle oxide polymer) and Avicel. After that Areosil is admixed to the obtained mixture and finally magnesium stearate is homogenously admixed. The obtained powder mixture is compressed into 300 mg oblong tablets.

The obtained tablets were coated with Opadry II HP coating (10% weight gain calculated on the weight of the core).

### Example 29: Film coated matrix tablets containing 75 mg of desvenlafaxine in form of sulphate salt

| Composition per tablet | |
|---|---|
| Desvenlafaxine sulphate | 34.3% |
| Metolose 90 SH 100,000 SR | 57.0% |
| Avicel pH101 | 6.0% |
| Talc | 2.2% |
| Magnesium stearate | 0.5% |

Desvenlafaxine sulphate is mixed with Metolose (hydroxypropylmethyl cellulose) and Avicel. After that talc is admixed to the obtained mixture and finally magnesium stearate is homogenously admixed. The obtained powder mixture is compressed into 300 mg oblong tablets.

The obtained tablets were coated with Opadry II HP coating (10% weight gain calculated on the weight of the core).

### Example 30: Film coated matrix tablets containing 75 mg of desvenlafaxine in form of sulphate salt

| Composition per tablet | |
|---|---|
| Desvenlafaxine sulphate | 34.3% |
| Metolose 90 SH 100,000 SR | 55.0% |
| Avicel pH101 | 6.0% |
| Pharmacoat 606 | 2.0% |
| Talc | 2.2% |
| Magnesium stearate | 0.5% |

Desvenlafaxine sulphate is mixed in the first step with Metolose (hydroxypropylmethyl cellulose) and Avicel. Pharmacoat is dissolved in purified water. Powder mixture obtained in first step is granulated with a solution of Pharmacoat in high sheare mixer/granulator. Obtained wet mass is put through the 1.0 mm sieve and dried in fluid bed dryer to the loss on drying 1.5-4% (Mettler halogen dryer at 85°C/20 min). After that talc and magnesium stearate are homogenously admixed to the dried granulate. The obtained powder mixture is compressed into 300 mg oblong tablets.

The obtained tablets were coated first with a separating layer (Opadry II white) and than with Eudragit EPO coating (the amount of applied coating is 3 mg/cm²).

| | |
|---|---|
| Eudragit EPO | 12.5% |
| Magnesium stearate | 4.4% |
| Stearic acid | 1.8% |
| Sodium lauryl sulphate | 1.3% |
| Purified water | 80.0% |

### Example 31: Film coated matrix tablets containing 75 mg of desvenlafaxine in form of sulphate salt

| | |
|---|---|
| Desvenlafaxine sulphate | 34.3% |
| Metolose 90 SH 100,000 SR | 54.0% |
| Avicel pH101 | 6.0% |
| Kollidon K90 | 3.0% |
| Talc | 2.2% |
| Magnesium stearate | 0.5% |

Desvenlafaxine sulphate is mixed in the first step with Metolose (hydroxypropylmethyl cellulose) Kollidon K90 (povidone) and Avicel. Powder mixture obtained in first step is granulated with a purified water in high sheare mixer/granulator. Obtained wet mass is put through the 1.0 mm sieve and dried in fluid bed dryer to the loss on drying 1.5-4% (Mettler halogen dryer at 85°C/20 min). After that talc and magnesium stearate are homogenously admixed to the dried granulate. The obtained powder mixture is compressed into 300 mg oblong tablets.

Obtained tablet cores are coated as described in Example 30.

### Example 32: matrix tablets containing 75 mg of desvenlafaxine in form of sulphate salt

| | |
|---|---|
| Desvenlafaxine sulphate | 34.3% |
| Metolose 90 SH 100,000 SR | 54.0% |
| Avicel pH101 | 6.0% |
| Kollidon K90 | 3.0% |
| Talc | 2.2% |
| Magnesium stearate | 0.5% |

Desvenlafaxine sulphate is mixed with part of Metolose (hydroxypropylmethyl cellulose) Kollidon K90 (povidone, Avicel and talc. The obtained mixture is dry granulated by roller compactor Hosokawa Bepex Pharmapaktor L200/50P with compaction force 20-60 kN. The obtained flakes are crushed on oscilating sieve (Frewit) using 1mm screen and mixed with the rest of excipients and compressed into tablets.

## Claims

1. A process for the preparation of a salt of O-desmethylvenlafaxine comprising
a) dissolving or suspending O-desmethylvenlafaxine in the form of its base in a liquid medium selected from the group of solvents and suspending agents;
b) contacting O-desmethylvenlafaxine with a pharmaceutically acceptable acid to form a pharmaceutically acceptable salt of O-desmethylvenlafaxine wherein the pharmaceutically acceptable acid is selected from the group consisting of HCl, HBr, H₂SO₄, H₃PO₄, acetic acid, adipic acid, galactaric acid, D-gluconic acid, glutamic acid, glutaric acid, glycolic acid, hippuric acid, lactic acid, oxalic acid, methanesulphonic acid, ethanesulphonic acid, *p*-toluenesulphonic acid, camphorsulphonic acid, benzenesulphonic acid, malonic acid, L-ascorbic acid, naphtalene-2-sulfonic acid and benzoic acid, optionally filtering the solution; and
c) separating the pharmaceutically acceptable salt of O-desmethylvenlafaxine from the liquid medium to recover the salt.

2. Process according to claim 1 wherein the pharmaceutically acceptable acid is selected from the group consisting of HCl, HBr, H₂SO₄, H₃PO₄, D-gluconic acid, glycolic acid, hippuric acid, oxalic acid, methanesulphonic acid, ethanesulphonic acid, *p*-toluenesulphonic acid, benzenesulphonic acid, malonic acid and naphtalene-2-sulfonic acid.

3. The process according to claim 1 or claim 2, wherein a solution is formed after the addition of the acid.

4. Process according to claim 1 or claim 2, wherein a solution is formed during or after heating to a temperature of up to the boiling point of the solvent used.

5. Process according to claim 4, wherein a solution is formed during or after heating under reflux conditions.

6. Process according to any of the preceding claims, wherein the salt is separated from the liquid medium by filtering at a temperature of up to the boiling point of the solvent or by filtering after cooling to room temperature.

7. Process according to any of the preceding claims, wherein the salt is recovered in a spray-drying process, lyophilization process, evaporation process or a process using super-critical fluid technology under super-critical conditions.

8. Process according to claim 1 or claim 2, comprising
a) suspending the base in a suspending agent at room temperature;
b) adding the pharmaceutically acceptable acid, optionally heating of the suspension at a temperature of up to the boiling point of the suspending agent and for a period of 0 to 24 h and subsequently cooling the suspension if necessary; and
c) separating the salt from the suspending agent to recover the salt.

9. Process according to any of the preceding claims, further comprising suspending the salt in a liquid organic medium after recovery of the salt in step c) of claim 1 or claim 8.

10. Process according to claim 9, wherein the liquid organic medium used is different from the liquid medium used in step a) of claim 1.

11. Process according to claim 9 or claim 10, wherein the organic medium is selected from the group consisting of ethers, esters and alcohols.

12. Process according to any of claims 8 to 11, wherein the organic medium is separated from the salt by filtering the suspension or evaporating the organic medium or by usding a spray-drying process, a lyophilization process, evaporation process or a process using super-critical fluid technology under super-critical conditions.

13. Process according to any of claims 8 to 12, wherein the additional suspending step is conducted at least two times.

14. Process according to any of the preceding claims wherein the solvent or suspending agent is selected from the group consisting of alcohols, ketones, tetrahydrofuran, dimethylformamide, dimethylacetamide, acetonitrile, water or mixtures thereof.

15. A process for the preparation of a pharmaceutical dosage form containing a salt of O-desmethylvenlafaxine wherein said salt is prepared according to a process as defined in any of the preceding claims and subsequently combined with suitable pharmaceutical excipients.

16. Process according to claim 15 wherein the salt of O-desmethylvenlafaxine is combined with the further excipients without separating the salt from the solvent and/or suspending agent used during the preparation of the salt.
